# EUROPEAN PATENT SPECIFICATION

(11) **EP 0 402 140 B1**
(45) Date of publication and mention of the grant of the patent: **07.02.1996**
(21) Application number: 90306200.8
(22) Date of filing: 07.06.1990
(51) Int. Cl.: A61F 13/38, A45D 40/26

(54) **Disposable swab for applying and removing cosmetics**
Wegwerftupfer zum Auftragen und Entfernen von kosmetischen Mitteln
Tampon applicateur à jeter pour appliquer et enlever des cosmétiques

(30) Priority: 08.06.1989 BR 8902707
(43) Date of publication of application: 12.12.1990
(73) Proprietor: JOHNSON & JOHNSON, New Brunswick, NJ 08933-7003 (US)
(72) Inventor: Anspach, Jean Michel Hubert, Campinas, SP (BR)
(74) Representative: Mercer, Christopher Paul

(56) References cited:
- EP-A- 0 363 533
- DE-U- 8 533 322
- US-A- 3 376 867
- US-A- 3 443 562
- US-A- 4 259 955

## Description

The present invention refers to swabs with absorbing ends, which can be adequately used in applying and removing cosmetics.

On the market there are swabs with absorbing cotton ends that are widely used for varying purposes such as babies ears, hygiene and cleaning tape-recorder magnetic heads. Such swabs have their ends wrapped in cotton wads, which is a material that has been used since long ago due to its softness and absorbency.

Such swabs with cotton ends have been used more and more as disposable make-up applicators/removers, because the applicators supplied with the cosmetics and consisting of rods with polyurethane foam ends do not have a good durability. This is due to the fact that polyurethane foam has a great affinity to oily materials such as cosmetics, by virtue of its small cells. Thus, after each application of make-up, such devices should be washed, which is not an easy task due to said small cells. As a result, such cosmetic applicators have a durability shorter than that of the cosmetic itself. Besides, swabs with cotton ends can be used in removing make-up, since they are disposable.

Therefore, it is easy to realize how practical the utilization of swabs with cotton ends is in applying and removing cosmetics. However, the user encounters some difficulty when using such swabs, mainly while removing the make-up, because cotton is hydrophilic rather than lipophilic, and therefore has no affinity to oily materials such as cosmetics.

Thus, the main objective of the present invention is to provide a swab with absorbing ends that can be used as a disposable cosmetic applicator and remover, without entailing any difficulty for the user.

German Utility Model DE-U-8533322 describes a swab consisting of consolidated cotton and/or viscose fibers and characterized by the inclusion of synthetic thermoplastic fibers distributed uniformly over the whole volume of the swab and bonded to the neighbouring fibers by melting, in the absence of pressure, of at least the surface of the thermoplastic fibers.

Earlier patent application EP-A-0363533, which was published after the priority date of the present application and which designates Germany, France and the United Kingdom, describes a disposable swab comprising a bar having a cotton-form group of fibers affixed to either of the ends of the bar, wherein the group of fibers include heat bondable bicomponent fibers each having a meltable layer on the surface thereof. The heat-bondable fibers are fused together by heating, thereby reducing the tendency of the group of fibers to unravel. The heat-bondable fibers may comprise a core of polypropylene or high density polyethylene surrounded by a lower melting point component such as low density polyethylene. The group of fibers may contain up to 100% of the heat-bondable fibers.

The present invention provides a disposable swab for applying and removing cosmetics, which comprises a rod having an absorbing material on at least one of its ends, characterized in that the absorbing material is a lipophilic microfibrous polyolefin wad.

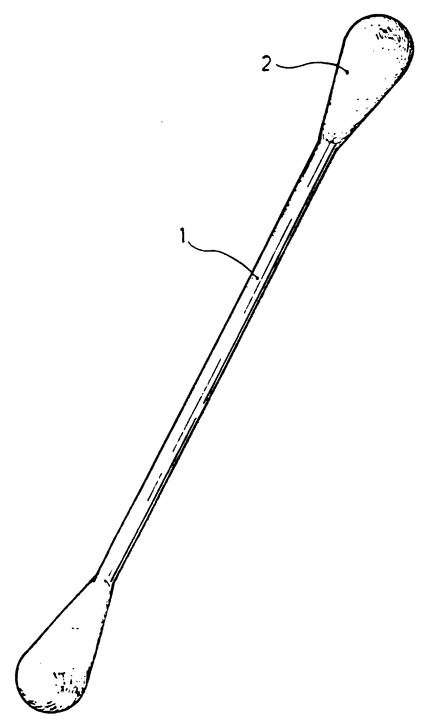

By referring to the accompanying drawing, it can be seen that the swab 1 has a general appearance quite similar to that of the usual rods with cotton ends of the type commercialized by Johnson & Johnson S.A. under the registered trademark "Cotonete". According to the teachings of the present invention, however, the swab 1 has at its ends portions 2 of lipophilic polyolefins such as polyethylene and polypropylene in the form of microfibers. Both the polyethylene and the polypropylene are acceptable lipophilic materials when they are in the form of microfibers.

Thus, the carded cotton wad that wraps the ends of the rods of "cotton swabs" is replaced by a "meltblown" polyolefin wad, which can be either polyethylene or polypropylene in spinnable form. However, from the manufacture standpoint, the best results have been obtained with non-spinnable polyethylene due to the low resistance of the wad obtained in this way, which facilitates the manufacture of the swabs with absorbing ends according to the invention.

In particular, the polymers with a flowability coefficient of approximately 30g/10min are the most recommendable. As example, can be mentioned the polyethylene Petrothene NA 608 (produced by Poliolefinas S/A) as well as the polypropylene Prolen Pex 7504 (produced by Polipropileno S/A).

The utilization of polyolefins in the form of microfibers allows the device of the present invention to be manufactured with the same machinery used in the manufacture of "cotton swabs", which substantially reduces the production costs thereof.

Besides, polyolefin can be treated so as to be hydrophilic too, thus giving way to a material that has all the characteristics of cotton (softness, absorbency), besides being lipophilic.

Obviously, the swab 1 can be made flexible to make the use thereof more comfortable and reliable, besides the fact that it may have the portion 2 either at one end or at both ends.

## Claims (Claims for the following Contracting State(s): ES, IT)

1. A disposable swab for applying and removing cosmetics, which comprises a rod having an absorbing material (2) on at least one of its ends, characterized in that the absorbing material (2) is a lipophilic microfibrous polyolefin wad.

2. A disposable swab according to claim 1, characterized in that the absorbing material (2) is a non-spinnable "meltblown" polyolefin wad.

3. A disposable swab according to claim 2, characterized in that the polyolefin has a flowability coefficient of approximately 30 g/10 min.

4. A disposable swab according to claim 3, characterized in that the polyolefin is polyethylene.

5. A disposable swab according to any one of the claims 1 to 4, characterized in that the rod is made of a flexible material.

6. A disposable swab according to claim 5, characterized in that the rod has absorbing material (2) at both ends.

7. A disposable swab according to claim 6, characterized in that the absorbing material (2) is hydrophilic too.

## Claims (Claims for the following Contracting State(s): GB)

1. A disposable swab for applying and removing cosmetics, which comprises a rod having an absorbing material (2) on at least one of its ends, characterized in that the absorbing material (2) is a lipophilic microfibrous polyolefin wad, and provided that the absorbing material does not include heat bondable bicomponent fibers each having a meltable layer on the surface thereof.

2. A disposable swab according to claim 1, characterized in that the absorbing material (2) is a non-spinnable "meltblown" polyolefin wad.

3. A disposable swab according to claim 2, characterized in that the polyolefin has a flowability coefficient of approximately 30 g/10 min.

4. A disposable swab according to claim 3, characterized in that the polyolefin is polyethylene.

5. A disposable swab according to any one of the claims 1 to 4, characterized in that the rod is made of a flexible material.

6. A disposable swab according to claim 5, characterized in that the rod has absorbing material (2) at both ends.

7. A disposable swab according to claim 6, characterized in that the absorbing material (2) is hydrophilic too.

## Patentansprüche (Patentansprüche für folgende(n) Vertragsstaat(en): ES, IT)

1. Wegwerftupfer zum Auftragen und Entfernen von kosmetischen Mitteln, der einen Stab aufweist, der ein absorbierendes Material (2) an mindestens einem seiner Enden hat, dadurch gekennzeichnet, daß das absorbierende Material (2) ein lipophiler, mikrofaseriger Polyolefin-Bausch ist.

2. Wegwerftupfer gemäß Anspruch 1, dadurch gekennzeichnet, daß das absorbierende Material (2) ein nicht-spinnbarer, "schmelzgeblasener" Polyolefin-Bausch ist.

3. Wegwerftupfer gemäß Anspruch 2, dadurch gekennzeichnet, daß das Polyolefin einen Fließfähigkeitskoeffizienten von ungefähr 30 g/10 min hat.

4. Wegwerftupfer gemäß Anspruch 3, dadurch gekennzeichnet, daß das Polyolefin Polyethylen ist.

5. Wegwerftupfer gemäß irgendeinem der Ansprüche 1 bis 4, dadurch gekennzeichnet, daß der Stab aus einem flexiblen Material besteht.

6. Wegwerftupfer gemäß Anspruch 5, dadurch gekennzeichnet, daß der Stab an beiden Enden absorbierendes Material (2) hat.

7. Wegwerftupfer gemäß Anspruch 6, dadurch gekennzeichnet, daß das absorbierende Material (2) auch hydrophil ist.

## Patentansprüche (Patentansprüche für folgende(n) Vertragsstaat(en): GB)

1. Wegwerftupfer zum Auftragen und Entfernen von kosmetischen Mitteln, der einen Stab aufweist, der ein absorbierendes Material (2) an mindestens einem seiner Enden hat, dadurch gekennzeichnet, daß das absorbierende Material (2) ein lipophiler, mikrofaseriger Polyolefin-Bausch ist, und vorausgesetzt, daß das absorbierende Material keine wärmeverschweißbaren Zweikomponentenfasern umfaßt, von denen jede eine schmelzbare Schicht auf ihrer Oberfläche hat.

2. Wegwerftupfer gemäß Anspruch 1, dadurch gekennzeichnet, daß das absorbierende Material (2) ein nicht-spinnbarer, "schmelzgeblasener" Polyolefin-Bausch ist.

3. Wegwerftupfer gemäß Anspruch 2, dadurch gekennzeichnet, daß das Polyolefin einen Fließfähigkeitskoeffizienten von ungefähr 30 g/10 min hat.

4. Wegwerftupfer gemäß Anspruch 3, dadurch gekennzeichnet, daß das Polyolefin Polyethylen ist.

5. Wegwerftupfer gemäß irgendeinem der Ansprüche 1 bis 4, dadurch gekennzeichnet, daß der Stab aus einem flexiblen Material besteht.

6. Wegwerftupfer gemäß Anspruch 5, dadurch gekennzeichnet, daß der Stab an beiden Enden absorbierendes Material (2) hat.

7. Wegwerftupfer gemäß Anspruch 6, dadurch gekennzeichnet, daß das absorbierende Material (2) auch hydrophil ist.

## Revendications (Revendications pour l'(les) Etat(s) contractant(s) suivant(s): ES, IT)

1. Tampon applicateur à jeter pour appliquer et enlever des cosmétiques, qui comprend une tige possédant une matière absorbante (2) à au moins une de ses extrémités, caractérisé en ce que la matière absorbante (2) est une ouate de polyoléfine microfibreuse lipophile.

2. Tampon applicateur à jeter selon la revendication 1, caractérisé en ce que la matière absorbante (2) est une ouate de polyoléfine non filable "soufflée en fusion".

3. Tampon applicateur à jeter selon la revendication 2, caractérisé en ce que la polyoléfine possède un coefficient de fluidité d'approximativement 30 g/10 min.

4. Tampon applicateur à jeter selon la revendication 3, caractérisé en ce que la polyoléfine est du polyéthylène.

5. Tampon applicateur à jeter selon l'une quelconque des revendications 1 à 4, caractérisé en ce que la tige est réalisée en un matériau flexible.

6. Tampon applicateur à jeter selon la revendication 5, caractérisé en ce que la tige possède une matière absorbante (2) à ses deux extrémités.

7. Tampon applicateur à jeter selon la revendication 6, caractérisé en ce que la matière absorbante (2) est également hydrophile.

## Revendications (Revendications pour l'(les) Etat(s) contractant(s) suivant(s): GB)

1. Tampon applicateur à jeter pour appliquer et enlever des cosmétiques, qui comprend une tige possédant une matière absorbante (2) à au moins une de ses extrémités, caractérisé en ce que la matière absorbante (2) est une ouate de polyoléfine microfibreuse lipophile, et avec cette réserve que la matière absorbante n'englobe pas des fibres thermocollables à deux composants, possédant chacune une couche fusible sur leur surface.

2. Tampon applicateur à jeter selon la revendication 1, caractérisé en ce que la matière absorbante (2) est une ouate de polyoléfine non filable "soufflée en fusion".

3. Tampon applicateur à jeter selon la revendication 2, caractérisé en ce que la polyoléfine possède un coefficient de fluidicité d'approximativement 30 g/10 min.

4. Tampon applicateur à jeter selon la revendication 3, caractérisé en ce que la polyoléfine est du polyéthylène.

5. Tampon applicateur à jeter selon l'une quelconque des revendications 1 à 4, caractérisé en ce que la tige est réalisée en un matériau flexible.

6. Tampon applicateur à jeter selon la revendication 5, caractérisé en ce que la tige possède une matière absorbante (2) à ses deux extrémités.

7. Tampon applicateur à jeter selon la revendication 6, caractérisé en ce que la matière absorbante (2) est également hydrophile.
